# EUROPEAN PATENT APPLICATION

(11) **EP 2 083 085 A1**
(43) Date of publication of application: **29.07.2009**
(21) Application number: 07806053.0
(22) Date of filing: 24.08.2007
(51) Int. Cl.: C12Q 1/02, C12N 15/09, C12Q 1/48, G01N 33/15, G01N 33/50

(54) **SCREENING METHOD FOR THERAPEUTIC AGENT FOR AMYOTROPHIC LATERAL SCLEROSIS**

(30) Priority: 04.09.2006 JP 2006239422
(71) Applicant: Hisamitsu Pharmaceutical Co., Inc., Tosu-shi, Saga 841-0017 (JP); Chiba-Prefecture, Chiba 260-8667 (JP)
(72) Inventor: NAKAGAWARA, Akira, Chiba-shi Chiba 260-0801 (JP); LIU, Tian-Ling, Chiba-shi Chiba 260-0801 (JP); KODA, Tadayuki, Tokyo 100-6221 (JP)
(74) Representative: Grünecker, Kinkeldey, Stockmair & Schwanhäusser Anwaltssozietät
(86) International application number: PCT/JP2007/066467
(87) International publication number: WO 2008/029646

(57) **Abstract**

The objective of the present invention is to provide methods of screening therapeutic agents for juvenile familial amyotrophic lateral sclerosis (ALS2). The invention provides a method of screening therapeutic agents for juvenile familial amyotrophic lateral sclerosis, comprising a step of assessing a substance that suppresses the expression of Tollip in cells as a therapeutic agent for juvenile familial amyotrophic lateral sclerosis; a method of screening therapeutic agents for juvenile familial amyotrophic lateral sclerosis, comprising a step of assessing a substance that promotes migration of Tollip in cells from the cytoplasm to the cell nucleus as a therapeutic agent for juvenile familial amyotrophic lateral sclerosis; and a method of screening therapeutic agents for juvenile familial amyotrophic lateral sclerosis, comprising a step of assessing a substance that inhibits the interaction between Tollip and IRAK-1 in cells as a therapeutic agent for juvenile familial amyotrophic lateral sclerosis.

## Description

### Technical Field

The present invention relates to methods of screening therapeutic agents for amyotrophic lateral sclerosis.

### Background Art

Amyotrophic lateral sclerosis (ALS) is a progressive neurodegenerative disease wherein the upper motor neurons that run from the cerebral cortex to the spinal cord, and the lower motor neurons that run from the spinal cord to muscles, are selectively damaged. Although it is considered to be a most severe nerve disease, no basic therapy has yet become available.

Depending on whether it is inherited, ALS is classified into sporadic ALS (SALS) and familial ALS (FALS). About 5 to 10% of all ALS cases are FALS. Investigation of the responsible gene of FALS is an important approach in elucidation of the causes of not only FALS but also SALS, which accounts for the majority of ALS cases, as the physical background of the motor neuron disorder and degeneration, which are common to all types ALS, has been clarified for FALS.

It is known that FALS has dominantly inherited ALS and recessively inherited ALS types. The SOD1 gene, which expresses Cu/Zn superoxide dismutase 1 (SOD1), has been identified as a responsible gene of ALS1, a type of ALS showing dominant inheritance (Non-Patent Document 1). However, the proportion of ALS 1 among all ALS cases is 2% or less, and moreover, the majority of SALS cases do not show the SOD1 gene mutation. For these reasons, it was expected that some genes other than SOD1 would be discovered as responsible genes of ALS.

Later, a deletion mutation was reported in the ALS2CR6 gene (subsequently renamed as ALS2), isolated from patients of juvenile recessively inherited ALS (ALS2), a type of recessively inherited ALS (Non-Patent Documents 2 and 3). The protein produced by the ALS2 gene has been named alsin. Alsin includes an amino acid sequence that is functionally and structurally very similar to those of GEF (guanine nucleotide exchange factor), an activation factor of GTPase, a signal transduction enzyme, it is possible that alsin is a new GTPase regulating factor; but the function of the protein has not yet been elucidated.

The 9 types of ALS2 gene mutations so far reported are all deletion mutations (Non-Patent Documents 2 to 4). For instance, a single-nucleotide deletion mutation in exon 3 (Tunisian; 138de1A) has been found in the Tunisian type, and a 2-nucleotide deletion mutation in exon 5 (Kuwaiti; 1425-1426de1AG) has been found in the Kuwait type. It is believed that because of these genes with deletion mutations, incomplete protein fragments of alsin with partially truncated C-terminal are translated, which impairs the basic function of alsin, and becomes the main cause of the onset of ALS2 (Non-Patent Document 2).

On the other hand, Tollip (Toll-interacting protein) is considered to be an adaptor protein of TLR (Toll-like receptor). There has been a report stating that force-expressed Tollip suppressed LPS-induced NFκB activity in the IL-1R/TLR signaling pathway (Non-Patent Document 5). There has been no report that Tollip binds to alsin.

Non-Patent Document 1: Rosen D. R. et al., Nature, 362: 59-62, 1993
Non-Patent Document 2: Yang Y. et al., Nat. Genet., 29: 103-104, 2001
Non-Patent Document 3: Hadano S. et al., Nat. Genet., 29: 166-173, 2001
Non-Patent Document 4: Kanekura K. et al., J. Biol. Chem., 279: 19247-19256, 2004
Non-Patent Document 5: Arnaud Didierlaurent et al., Molecular and Cellular Biology, 26: 735-742, 2006

### Disclosure of the Invention

### Problems to be Solved by the Invention

As described above, although the ALS2 gene has been identified as a prominent responsible gene for the elucidation of the causes of ALS, the functions of alsin, the protein produced by this gene, are not yet known, and the studies have not reached the stage of elucidation of the mechanism of onset of ALS, or the development of therapeutic methods.

One of the objectives of the present invention is the elucidation of the intracellular functions of alsin and its molecular mechanism in signal transduction, and another objective is providing a method of screening therapeutic agents for juvenile familial amyotrophic lateral sclerosis (ALS2) based on the molecular mechanism.

### Means for Solving the Problems

After painstaking investigations to achieve the aforementioned objectives, the present inventors have found out that Tollip is the protein that binds to the wild type alsin, the protein produced by the ALS2 gene, that the MORN motif domain of alsin is essential for binding of alsin to Tollip, further that mutant alsin with deleted MORN motif could no longer bind to Tollip, and therefore, could not suppress Tollip-induced cell death, and that this was one of the causes of the onset of juvenile familial amyotrophic lateral sclerosis. The present inventors further have found out that alsin was present only in the cytoplasm, that Tollip was present in both the cytoplasm and the cell nucleus, that Tollip migrated from the cytoplasm to the cell nucleus under mediation by TNF-α, and that Tollip induced cell death by binding to IRAK-1 in TNF signaling, which led to perfection of the present invention.

In other words, the present invention firstly provides a method of screening therapeutic agents for juvenile familial amyotrophic lateral sclerosis, comprising a step of assessing a substance that suppresses the expression of Tollip in cells as a therapeutic agent for juvenile familial amyotrophic lateral sclerosis. This method of screening is based on the molecular mechanism of the onset of juvenile familial amyotrophic lateral sclerosis, in other words, the molecular mechanism wherein a deletion-mutated alsin is unable to prevent Tollip-induced cell death because of its inability to bind to Tollip. As this molecular mechanism is a new discovery made by the present inventors, therapeutic agents for juvenile familial amyotrophic lateral sclerosis having an action mechanism that is completely different from what is known so far can be selected by this screening method.

The present invention provides a method of screening therapeutic agents for juvenile familial amyotrophic lateral sclerosis, comprising steps of: culturing cells expressing Tollip under the conditions of the presence and absence of a test substance; measuring the level of Tollip expression in the cells cultured under the different conditions; and assessing the test substance as a therapeutic agent for juvenile familial amyotrophic lateral sclerosis if the level of Tollip expression in the cells cultured in the presence of the test substance is less than in the cells cultured in the absence of the test substance. Therapeutic agents for juvenile familial amyotrophic lateral sclerosis having an action mechanism that is completely different from what is known so far can be selected by this screening method.

The present invention further provides a method of screening therapeutic agents for juvenile familial amyotrophic lateral sclerosis, comprising a step of assessing a substance that promotes migration of Tollip in cells from the cytoplasm to the cell nucleus as a therapeutic agent for juvenile familial amyotrophic lateral sclerosis. This screening method is based on the molecular mechanism of the onset of juvenile familial amyotrophic lateral sclerosis, i.e., the molecular mechanism wherein a deletion mutated alsin is unable to prevent Tollip-induced cell death because of its inability to bind to Tollip, and the finding that alsin is present only in the cytoplasm, whereas Tollip is present both in the cytoplasm and the cell nucleus. As this molecular mechanism and the above-mentioned finding are new discoveries made by the present inventors, therapeutic agents for juvenile familial amyotrophic lateral sclerosis having an action mechanism that is completely different from what is known so far can be selected by this screening method.

The present invention provides a method of screening therapeutic agents for juvenile familial amyotrophic lateral sclerosis, comprising steps of: culturing cells that express Tollip under the conditions of the presence and absence of a test substance; and assessing the test substance as a therapeutic agent for juvenile familial amyotrophic lateral sclerosis if the ratio of the amount of Tollip present in the cell nuclei, with respect to the total of the amount of Tollip present in the cytoplasm and the amount of Tollip present in the cell nuclei, is higher in the cells cultured in the presence of the test substance than in the cells cultured in the absence of the test substance. Therapeutic agents for juvenile familial amyotrophic lateral sclerosis having an action mechanism that is completely different from what is known so far can be selected by this screening method.

The present invention provides a method of screening therapeutic agents for juvenile familial amyotrophic lateral sclerosis, comprising a step of assessing a substance that inhibits the interaction between Tollip and IRAK-1 in cells as a therapeutic agent for juvenile familial amyotrophic lateral sclerosis. This screening method is based on the molecular mechanism of the onset of juvenile familial amyotrophic lateral sclerosis, i.e., the molecular mechanism wherein the deletion-mutated alsin is unable to prevent Tollip-induced cell death because of its inability to bind to Tollip, and the finding that in TNF-signaling Tollip binds to IRAK-1 to induce cell death. As this molecular mechanism and the above-mentioned finding are new discoveries made by the present inventors, therapeutic agents for juvenile familial amyotrophic lateral sclerosis having an action mechanism that is completely different from what is known so far can be selected by this screening method.

The present invention provides a method of screening therapeutic agents for juvenile familial amyotrophic lateral sclerosis, comprising steps of: culturing cells that express Tollip and IRAK-1 under the conditions of the presence and absence of a test substance; measuring the interaction between Tollip and IRAK-1 in the cells cultured under the different conditions; and assessing the test substance as a therapeutic agent for juvenile familial amyotrophic lateral sclerosis if the interaction between Tollip and IRAK-1 in the cells cultured in the presence of the test substance is weaker than in the cells cultured in the absence of the test substance. Therapeutic agents for juvenile familial amyotrophic lateral sclerosis having an action mechanism that is completely different from what is known so far can be selected by this screening method.

In the methods of screening therapeutic agents for juvenile familial amyotrophic lateral sclerosis of the present invention, it is preferable to assume that the ALS2 gene is a responsible gene for the juvenile familial amyotrophic lateral sclerosis. Based on new findings made by the present inventors, it is believed that cell death induced by Tollip is one of the causes for the onset of juvenile familial amyotrophic lateral sclerosis. Therefore, although identifying a responsible gene is not essential, if ALS2 were indeed a responsible gene, therapeutic agents for juvenile familial amyotrophic lateral sclerosis can be selected with higher certainty by these screening methods.

### Effects of the Invention

Therapeutic agents for juvenile familial amyotrophic lateral sclerosis having an action mechanism that is completely different from what is known so far can be selected by the screening methods of the present invention. It is believed that therapeutic agents selected by the screening methods of the present invention would be promising new therapeutic agents for juvenile familial amyotrophic lateral sclerosis.

### Brief Description of the Drawings

Figure 1 is a diagrammatic image of the domains of alsin and Tollip;
Figure 2 (A) is a diagrammatic image of the ALS2CR6-pAS2-1 vector, the bait vector used in the yeast two-hybrid system, (B) is a diagrammatic image of the cDNA library-pACT-2 vector, the prey vector used in the yeast two-hybrid system, and (C) is a diagrammatic image of interaction between alsin and Tollip in the yeast two-hybrid system;
Figure 3 is a diagrammatic image of expression vectors with different tags used for immunoprecipitation;
Figure 4 is a diagram showing the results of immunoprecipitation in Example 2, (A) is the results of western blotting carried out to check for complex formation between full-length ALS2 and Tollip, (B) is the result of western blotting for checking complex formation between respective domains of ALS2 and Tollip, and (C) is the result of western blotting for checking complex formation between the MORN domain of ALS2 and Tollip;
Figure 5 is a diagram showing the results of trypan blue staining carried out for checking the effect of alsin and Tollip on cell death of NSC34 cells;
Figure 6 (A) is a diagram showing the results of FAC-scanning carried out for checking the effect of alsin and Tollip on cell death of HEK293 cells, and (B) shows the results of western blotting carried out for checking the levels of alsin and Tollip expression in HEK293 cells;
Figure 7 is a diagram showing the results of flow cytometry carried out to examine the effect of alsin and Tollip on cell death of HEK293 cells, (A), (B), (C), and (D) are, respectively, results obtained with cells transfected with the empty vector, full-length ASL2 expression vector, Tollip expression vector, and full-length ASL2 expression vector + Tollip expression vector;
Figure 8 is a diagram showing the results of trypan blue staining carried out to examine the effect of alsin, its respective domains, and Tollip on cell death of NSC34 cells;
Figure 9 (A) is a diagram showing the results of luciferase assay on the induction of NFκB activity caused by treatment with 10 ng/mL TNF-α for different durations, (B) is a diagram showing the results of luciferase assay on the induction of NFκB activity caused by treatment with different concentrations of TNF-α for 1 h, and (C) is a diagram showing the results of checking the level of induction of NFκB activity by TNF-α in the cytoplasm (C) and the cell nucleus (N);

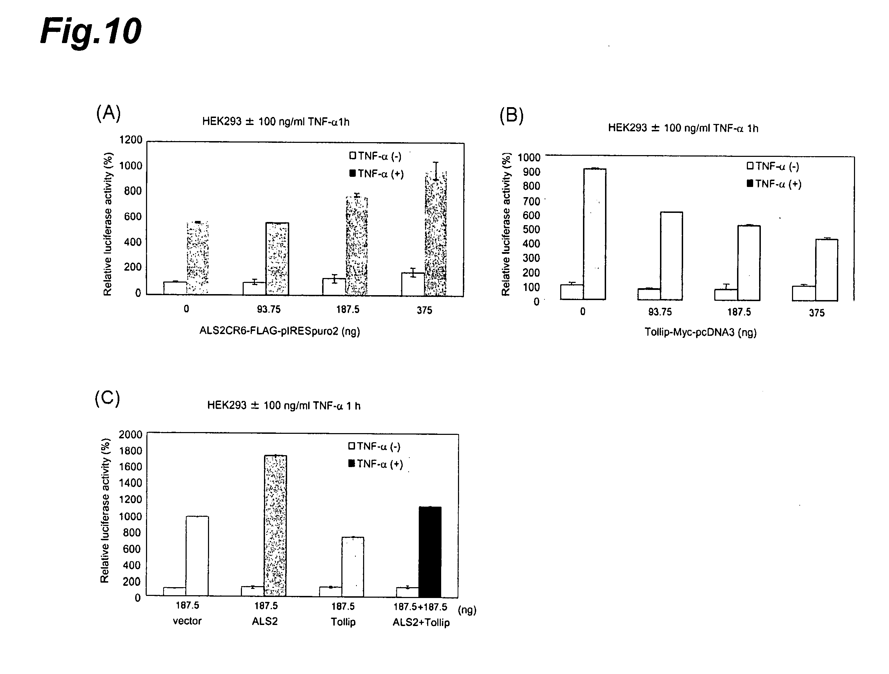

Figure 10 (A) is a diagram showing the results of checking the level of induction of NFκB activity, with and without 1 h treatment with 100 ng/mL TNF-α, after transfection with different amounts of ALS2, (B) is a diagram showing the results of checking the level of induction of NFκB activity with and without 1 h treatment with 100 ng/mL TNF-α, after transfection with different amounts of Tollip, and (C) is a diagram showing the results of luciferase assay for checking NFκB activity in HEK293 cells transfected with ALS2 alone, Tollip alone, or both ALS2 and Tollip, with and without 1 h treatment with 100 ng/mL TNF-α;
Figure 11 is a diagram showing the results of examination, by immunoprecipitation, of the interaction between Tollip and IRAK-1, (A) and (B) respectively show results of immunoblotting (IB) using anti-IRAK-1 antibody and anti-Myc antibody;
Figure 12 (A) and (B) are respectively, the results of western blotting for examining the intracellular localization of alsin and Tollip, wherein the cell nucleus is represented by N and the cytoplasm by C; and
Figure 13 is a diagram showing the results of western blotting carried out at different time points to examine the changes in the intracellular localization of Tollip caused by treatment with TNF-α, wherein the cell nucleus is represented by N and the cytoplasm by C.

### Best Modes for Carrying Out the Invention

The preferred embodiments of the present invention are described below in detail.

### <Gene and Protein>

Firstly, we shall describe the genes ALS2, Tollip, and IRAK-1, and the proteins produced by them, which are related to the screening methods of the present invention. In the present description, sometimes a gene and the protein produced by the gene have the same name, but which is being referred to should be obvious to persons skilled in the art, without specific mention of whether it is a gene or protein. The same applies also when the disease name and the name of the responsible gene are the same.

### (ALS2 gene and alsin)

The ALS2 gene, also known as ALS2CR6, is believed to be a responsible gene of juvenile familial amyotrophic lateral sclerosis. The human ALS2 gene is basically present in the chromosome 2q33, and has a total length of about 80.3 bp with 34 exons. The nucleotide sequence of ALS2 excluding the intron has been identified (total length 6470 kb) and has been registered as NM_020919 with GenBank (trademark). The part of ALS2 translated into amino acids (CDS) is 4974 bp (200 to 5173).

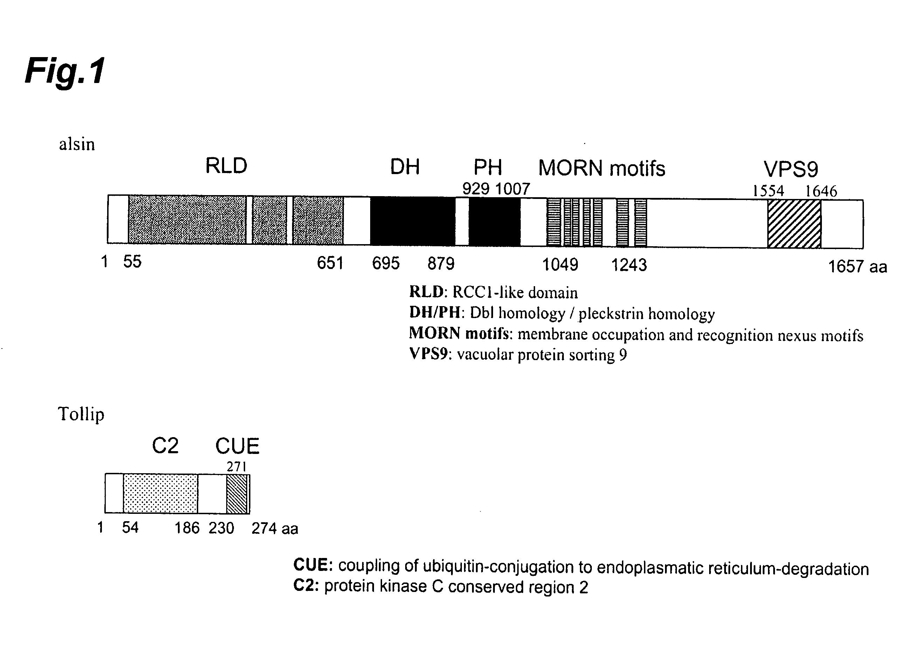

The protein product of human ALS2 is named as alsin. Alsin has 1657 amino acids, and 4 domains: RLD, DH-PH, MORN motifs, and VPS9, in that order, starting from the N-terminus (Figure 1).

### (Tollip gene and its protein product)

The human Tollip gene has a total length of 3615 bp, and has been registered as NM_019009 with GenBank (trademark). CDS of Tollip is 825 bp (112 to 936). Tollip is a protein having 274 amino acid residues and has two domains, C2 and CUE, in that order, starting from the N-terminus (Figure 1).

### (IRAK-1 gene and its protein product)

The human IRAK-1 (interleukin-1 receptor-associated kinase 1) gene has a total length 3569 bp and is registered as NM_001569 with GenBank (trademark). The CDS of IRAK-1 has 2139 bp (80 to 2218). IRAK-1 is a protein having 712 amino acid residues.

### <Screening methods>

We shall next describe the methods of screening therapeutic agents for juvenile familial amyotrophic lateral sclerosis in the present embodiment of the invention. The screening methods of the present invention include a first screening method wherein the suppression of Tollip expression in cells is used as the index, a second screening method wherein the promotion of Tollip migration in cells from the cytoplasm to the cell nucleus is used as the index, and a third screening method wherein the inhibition of the interaction between Tollip and IRAK-1 in cells is used as the index.

### (First screening method)

Firstly, we shall describe the first screening method wherein the suppression of Tollip expression in cells is used as the index. The first screening method comprises a step of assessing a substance that suppresses Tollip expression in the cells as a therapeutic agent for juvenile familial amyotrophic lateral sclerosis. Here "suppression of Tollip expression" means a reduction in the expression of the transcription product mRNA of the Tollip gene and/or its translation product protein (hereinafter referred to as the "level of Tollip expression", with reference to the first screening method).

In the first screening method, if a certain test substance can reduce the amount of the transcription product mRNA of the Tollip gene and/or its translation product protein, that test substance is assessed as a therapeutic agent for juvenile familial amyotrophic lateral sclerosis. Any system known to persons skilled in the art that can measure the amount of mRNA or protein expressed can be used for measuring the level of Tollip expression. Specific examples of methods of measuring the amount of mRNA expressed include quantitative RT-PCR, real time quantitative RT-PCR, quantitative northern blotting, and quantitative ribonuclease protection analysis, and examples of methods of measuring the amount of protein expressed include quantitative western blotting, ELISA, etc. In this case, the level of Tollip expression can be standardized using, as control, the expression of GADPH, a housekeeping gene, and the mRNA and/or protein of β-action and the like. It is preferable that the Tollip is of mammalian origin, more preferably of human origin.

In the first screening method, it is preferable that the culturing step, wherein cells expressing Tollip are cultured, is done under the conditions of the presence and the absence of the test substance. Here, "cells expressing Tollip" means cells having an expressible Tollip gene or its cDNA, and moreover, can transcribe and translate the Tollip gene. In particular, cells transfected with an expression plasmid wherein Tollip cDNA has been inserted in an expressible state is preferably used. The Tollip gene is a known gene. Therefore, the Tollip gene or its cDNA can be obtained by selective amplification using an experiment system known to persons skilled in the art, such as RT-PCR or PCR. Besides, transfection and cell culturing are known techniques for persons skilled in the art. A cell culturing time sufficient for transcription and expression of the Tollip gene is adequate. For example, the culturing time is 12 to 48 h, although it depends on the type of cells and the promoters of the plasmid used.

Next, the step of measuring the level of Tollip expression in the cells cultured under the different conditions is carried out. As for the method of measuring the level of Tollip expression, any measuring system known to a person skilled in the art, which can measure the amount of mRNA or protein expressed, can be used. Specific examples of the methods have been mentioned above.

The last is the step of assessing the test substance as a therapeutic agent for juvenile familial amyotrophic lateral sclerosis if the level of Tollip expression in the cells cultured in the presence of the test substance is less than in the cells cultured in the absence of the test substance. A substance that has a suppressive action on the expression of Tollip in the cells is finally selected through this assessment step. Substances having such action have the potential to alleviate the symptoms of juvenile familial amyotrophic lateral sclerosis, especially the symptoms like motor disorder arising from motor neuron damage or degeneration, or to delay or stop the advancement of such symptoms.

### (Second screening method)

Next, we shall describe the second screening method wherein the promotion of migration of Tollip from the cytoplasm to the cell nucleus in the cells is used as the index. The second screening method comprises a step of assessing a substance that promotes the migration of Tollip from the cytoplasm to the cell nucleus as a therapeutic agent for juvenile familial amyotrophic lateral sclerosis.

Here, "migration of Tollip from the cytoplasm to the cell nucleus" means decrease in the amount of mRNA, which is the transcription product of the Tollip gene, and/or the protein, which is its translation product, in the cytoplasm and increase in their amounts in the cell nucleus. The "amount of Tollip present" means the level of Tollip expressed in a location. In other words, it is the expressed amount of mRNA, which is the transcription product of the Tollip gene, and/or the protein, which is its translation product, in the location (hereinafter referred to as the "amount of Tollip present", with reference to the second screening method). For measuring the amount of Tollip present, the methods in the first screening method used for measuring the level of Tollip expressed can be used. Therefore, their description is omitted here. It is preferable that the Tollip is of mammalian origin, preferably human origin.

It is preferable that the second screening method firstly comprises a step of culturing Tollip-expressing cells under the different conditions of the presence and the absence of the test substance. This culturing step is the same as the culturing step used in the first screening method. Therefore, its description is omitted here.

Next, the step of measuring the amount of Tollip present in the cytoplasm and the cell nuclei of cells cultured under the different conditions is carried out. For this purpose, the cells need to be fractionated first. Any experiment system known to persons skilled in the art, which can fractionate the cells into cytoplasmic and nuclear fractions, may be used for fractionating the cells. For example, one of the several commercially available cell fractionation kits can be used.

Lastly, a step of assessing the test substance as a therapeutic agent for juvenile familial amyotrophic lateral sclerosis if the ratio of the amount of Tollip present in the cell nuclei, with respect to the total of the amount of Tollip present in the cytoplasm and in the cell nuclei, is higher in cells cultured in the presence of the test substance than in cells cultured in the absence of the test substance, is carried out. Substances that have a promoting effect on the migration of Tollip from the cytoplasm to the cell nucleus in the cells can be finally selected through this assessment step. Substances having such an effect have the potential to alleviate the symptoms of juvenile familial amyotrophic lateral sclerosis, especially the symptoms like motor disorder arising from motor neuron damage or degeneration, or to delay or stop the advancement of such symptoms.

### (Third screening method)

Finally, we shall describe the third screening method wherein the inhibition of the interaction between Tollip and IRAK-1 in cells is used as the index. The third screening method comprises a step of assessing a substance that inhibits the interaction between Tollip and IRAK-1 in the cells as a therapeutic agent for juvenile familial amyotrophic lateral sclerosis. Here, "interaction between Tollip and IRAK-1" means mainly the binding of Tollip protein to IRAK-1 protein. "Inhibition of the interaction" here means the reduction of the amount of binding of the Tollip protein to the IRAK-1 protein, or the reduction of the strength of such binding, irrespective the mechanisms involved.

Any system for measuring the interaction between proteins, known to persons skilled in the art, such as immunoprecipitation, can be used for the third screening method. More specifically, the cultured cells are first ground, and cell lysate is prepared. Immunoprecipitation is then carried out by adding an antibody against one of the molecules, either Tollip or IRAK-1, to the cell lysate thus prepared, and the precipitate (containing the complex of Tollip and IRAK-1) thus obtained is subjected to an immunological technique (such as immunoblotting, etc.) using an antibody against the other molecule, to detect and quantify the Tollip-IRAK-1 complex and thus measure the interaction between the two proteins. It is preferable that the Tollip and IRAK-1 are of mammalian origin, more preferably of human origin.

In the third screening method, it is preferable to first carry out the step of culturing the cells that express Tollip and IRAK-1, under the conditions of the presence and absence of the test substance. As for cells that express Tollip and IRAK-1, cells that express both of them, cells that express either one and are transfected with the other so that they express both, or cells that do not express either one of them and are co-transfected with both, may be used. Such cells are then cultured under the conditions of the presence and the absence of the test compound. The suitable culturing time is any culturing time that allows interaction of Tollip and IRAK-1, and it differs depending on the cells used. A cell culturing time sufficient for interaction of Tollip and IRAK-1 is adequate, and this differs depending on the cells used. For example, in the case of cells co-transfected with Tollip and IRAK-1, this time is about 12 to 48 h.

Next, the step of measuring the interaction of Tollip and IRAK-1 in the cells cultured under the different conditions is carried out. The specific methods of measurement have been described above, and therefore, their description is omitted here.

Finally, the step, wherein the test substance is assessed as a therapeutic agent for juvenile familial amyotrophic lateral sclerosis if the interaction between Tollip and IRAK-1 in the cells cultured in the presence of the test substance is weaker than in the cells cultured in the absence of the test substance, is carried out. Substances that have an inhibitory action on the interaction between Tollip and IRAK-1 in the cells can be finally selected through this assessment step. Substances having such action have the potential to alleviate the symptoms of juvenile familial amyotrophic lateral sclerosis, especially the symptoms like motor disorder arising from motor neuron damage or degeneration, or to delay or stop the advancement of such symptoms.

The present invention is described more specifically below, citing examples. However, the scope of the present invention is not restricted in any way by these examples.

### Examples

### (Preparation of ALS2 cDNA)

pBluescript II SK (+) KIAA1563 (FH20460) vector containing a part of the ALS2 gene was procured from Kazusa DNA Research Institute. The remaining 5' portion was synthesized by polymerase chain reaction (PCR). PCR was carried out following the method of Patel S et al., J. Immunol. Methods, 205: 157-161, 1997. A human brain cDNA library (BD Matchmaker Library; Clontech) was used as the template. Forward: 5'-atggggtaccggttgtcagtt-3' (Sequence No. 1) and Reverse: 5'-ttgaagcctaggcagaacatc-3' (Sequence No. 2) were used as the oligonucleotide primers.

Next, the synthesized 5' portion was treated with the restriction enzymes KpnI and AvrII, and inserted into the KpnI and AvrII sites of the pBluescript II SK (+) KIAA1563 vector that had been similarly treated. After that, the vector was treated with the restriction enzymes KpnI and NotI to obtain cDNA of the full-length ALS2. This full-length ALS2 was integrated into with a pBS2 vector (Toyobo).

Then, using the ALS2/pBS2 obtained as a template, cDNA corresponding to respective domains of alsin: RLD (200 to 2215), DH/PH (2207 to 3274), MORN (3269 to 3997), and VPS9 (4466 to 5170), were amplified by PCR and each was integrated into a pBS2 vector.

The nucleotide sequence of cDNA of the full-length ALS2 and each of the domains was verified using an ABI PRISM 377 DNA sequencer (manufactured by Perkin Elmer), following the protocol provided by the manufacturer. The results showed that the cDNA of the full-length ALS2 has 4974 bp, which matched with the nucleotide sequence 200 to 5173 of the NM_020919 gene registered with GenBank (trademark), and that the cDNA of each domain also matched with the nucleotide sequence of the respective domain.

### (Example 1 Screening of proteins that interact with alsin)

The screening of proteins that interact with alsin was carried out using the yeast two-hybrid system. The two-hybrid method was implemented following the procedure described by Miyazaki, K et al., J. Biol. Chem., 279: 11327-11335, 2004.

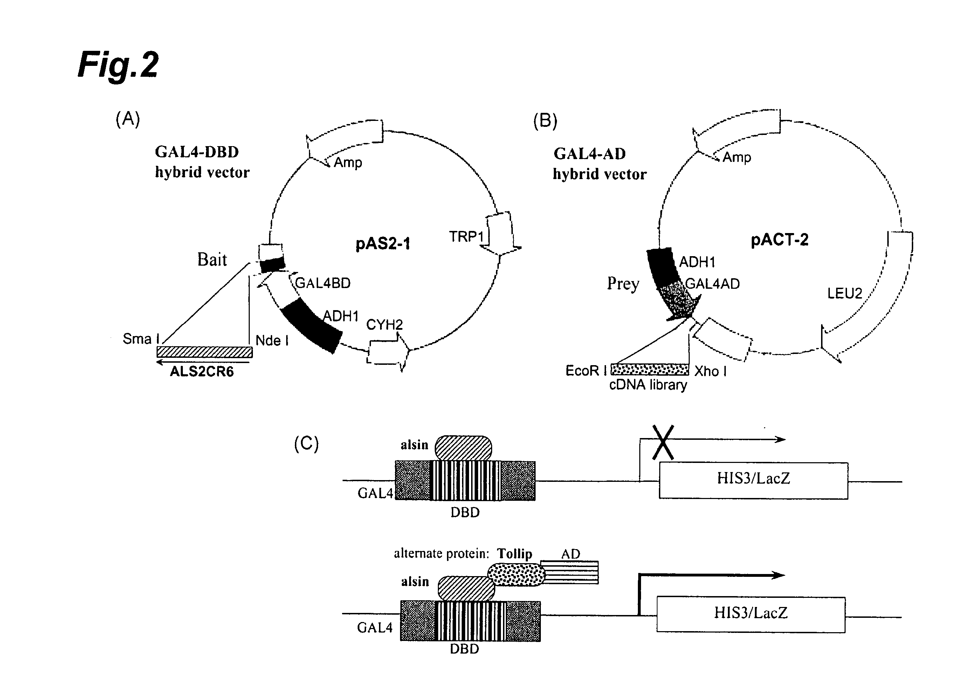

The pAS2-1 vectors (Clontech) into which the cDNA of the full-length ALS2 or one of the ALS2 domains had been inserted at the DNA binding domain (DBD) of the yeast transcription factor GAL4 were used as the bait vectors, and the pACT-2 vectors (Clontech) into which various cDNA libraries had been inserted at the yeast transcription activating domain (AD) were used as the prey vectors (Figure 2 (A) and (B)).

The pAS2-1 vector has the TRP1 gene, and therefore, can grow in a tryptophan-free medium (Trp(-)). On the other hand, pACT-2 has the LEU2 gene and can grow in a leucine-free medium (Leu(-)). When a complex is formed by interaction of the two types of fusion protein (two-hybrid) expressed, the yeast cell reporter gene His3/LacZ is expressed and growth occurs even in a histidine-free medium (His(-)). Besides, β-galactosidase is also activated (Figure 2 (C)).

Both the bait vectors and the prey vectors were transfected into yeast cells CG-1945 (Clontech) by the lithium acetate method, which were then inoculated on Trp(-)/Leu(-)/His(-) plates to obtain His-resistant yeast. After culturing for 7 days at 30°C, the colonies obtained were scooped out and transferred to fresh Trp(-)/Leu(-)/His(-) plates, and then transferred on to nitrocellulose membranes. To detect activation of β-galactosidase, which appears only when a complex is formed, the colonies on the membranes were reacted with X-gel, and colonies that turned blue because of the activation of β-galactosidase were designated as positive colonies. The nucleotide sequence of the gene that had been inserted into the prey vector of the positive colonies was identified by sequencing and compared with a database. As a result, the protein that binds to alsin was identified as Tollip.

### (Example 2 Identification of the alsin domain that binds to Tollip)

Immunoprecipitation was carried out to identify the alsin domain that binds to Tollip. The immunoprecipitation was done following the method described by Miyazaki, K. et al., J. Biol. Chem., 279: 11327-11335, 2004.

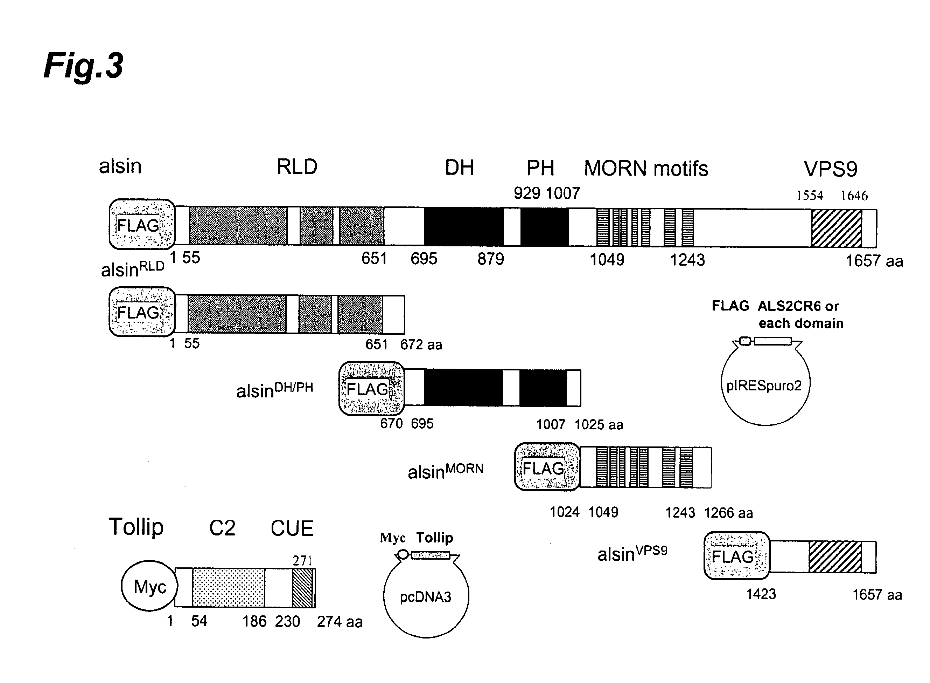

Firstly, as shown in Figure 3, pIRESpuro2 (Clontech) into which cDNA of the full-length ALS2 or one of the respective domains was inserted downstream of a FLAG tag (5'-gactacaaggacgacgatgacaag-3'; Sequence No. 3), in such a way that it can form fusion protein, was prepared. A pcDNA3 (Invitrogen) vector, into which cDNA of Tollip was inserted downstream of a Myc tag (5'-gaacaaaaactcatctcagaagaggatctg-3'; Sequence No. 4) so that fusion protein can be formed, was also prepared.

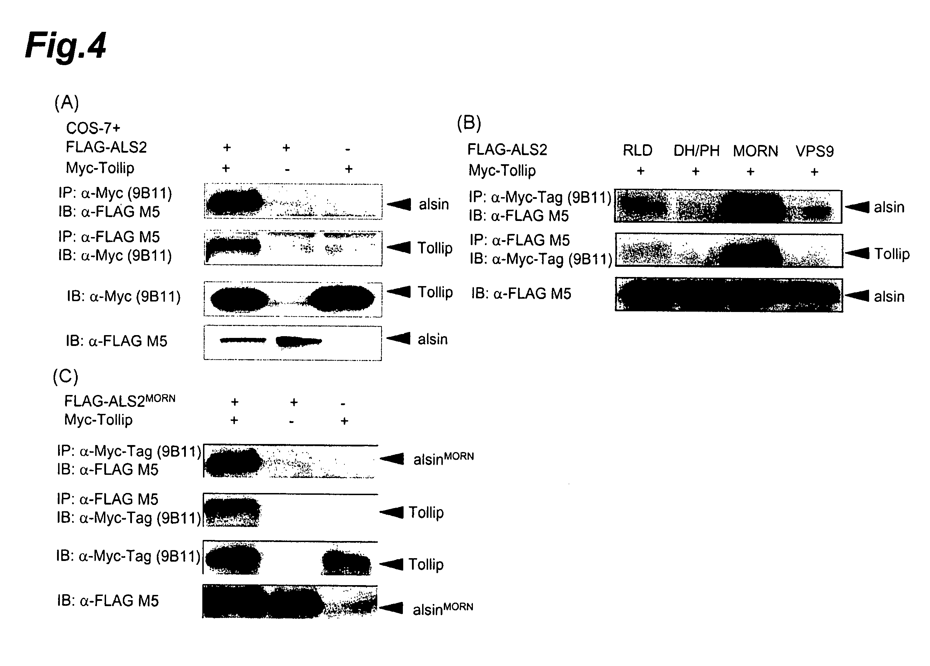

COS-7 cells were co-transfected with the two plasmids thus obtained, using Lipofectamin 2000. After culturing the cells for 48 h, they were lysed. The cell lysate was then subjected to immunoprecipitation (IP) using a Protein G Sepharose 4 Fast Flow (Amersham Biosciences) and anti-FLAG M5 antibody. After that, immunostaining (IB) was done using anti-Myc antibody. Besides this, after immunoprecipitation using anti-Myc antibody, confirmation was done by immunostaining with an anti-FLAG M5 antibody. Results are shown in Figure 4.

Figure 4 (A) shows the result of checking the interaction between the full-length ALS2 and Tollip, which confirmed that the full-length ALS2 and Tollip formed a complex. Figure 4 (B) shows the results of checking the interactions between different domains of ALS2 and Tollip. The results showed that of the four domains, only MORN could form a complex with Tollip. Figure 4 (C) shows the results of checking the interaction between the MORN domain and Tollip, which confirmed that the MORN domain and Tollip formed a complex. The above results confirmed that the MORN domain of ALS2 specifically binds to Tollip.

### (Example 3 Suppression of Tollip-induced cell death by full-length ALS2)

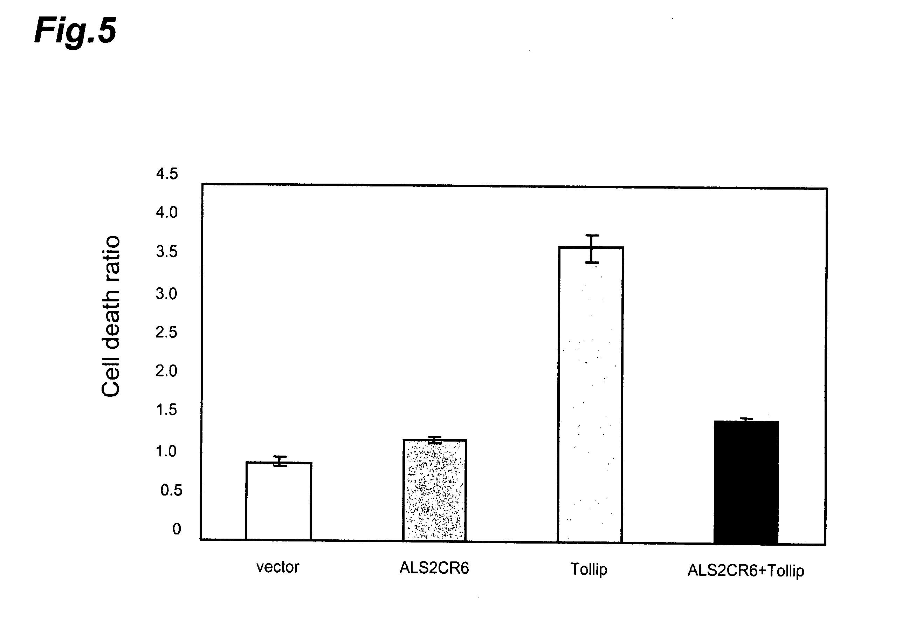

NSC34 cells were transfected with a full-length ALS2 expressing vector and a Tollip expressing vector separately or together. 72 h after that, the cells were stained with trypan blue to check for cell death. The NSC34 cells were procured from Neil Cashman's Laboratory (Centre for Research in Neurodegenerative Diseases, University of Toronto, Canada). The results of the trypan blue staining are given in Figure 5.

Figure 5 confirms that almost no cell death occurred when NSC34 cells were transfected with full-length ALS2. However, cell death occurred in about 40% of the NSC34 cells transfected with Tollip alone. On the other hand, suppression of Tollip-induced cell death in cells where alsin and Tollip were co-expressed was also confirmed. An empty vector was used as control.

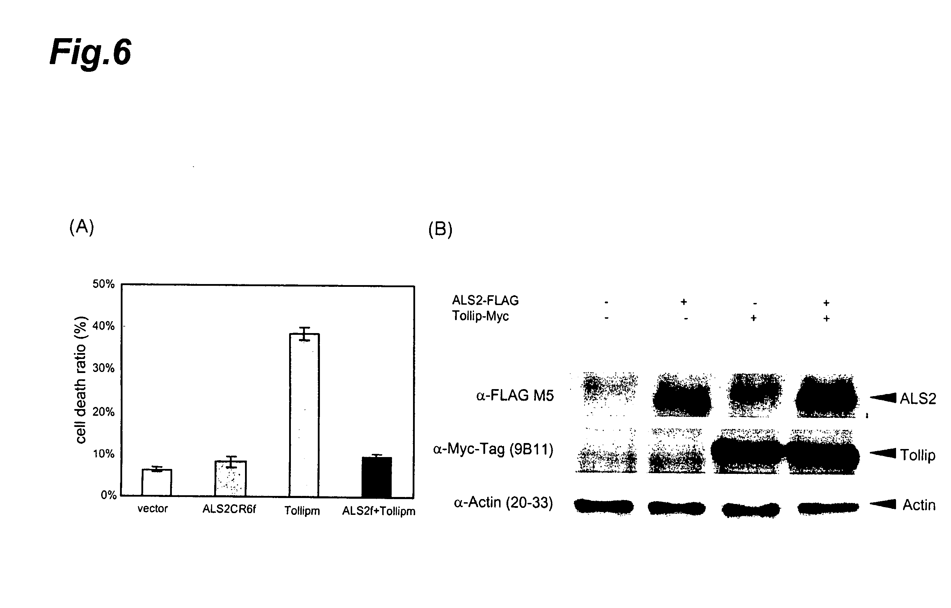

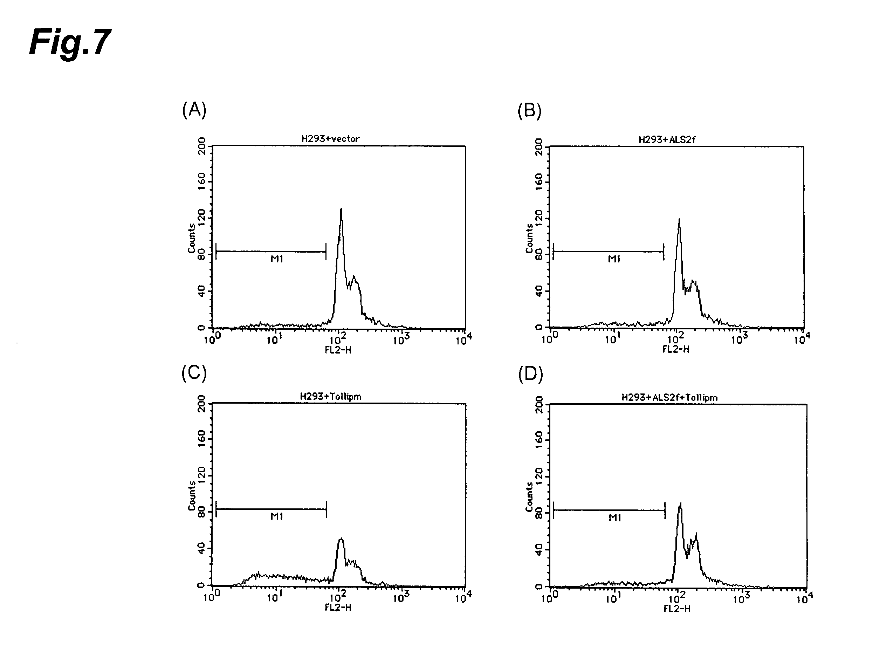

Experiments were carried out also with HEK293 cells in place of the NSC34 cells. HEK293 cells that had been transfected with full-length ALS2 alone, Tollip alone, full-length ALS2 + Tollip, or a control empty vector were stained with 100 µg/mL of a fluorescent dye (PI), and flow cytometry carried out with FACScan (BECTON DICKINSON), and cell death was examined. The results are given in Figure 6 (A) and Figure 7.

Furthermore, the amounts of alsin and Tollip expressed in HEK293 cells that had been transfected with full-length ALS2 alone, Tollip alone, full-length ALS2 + Tollip, or a control empty vector were measured by western blotting (WB). In the WB, mouse anti-FLAG IgG antibody, or mouse anti-Myc IgG antibody was used as the primary antibody. Rabbit anti-actin IgG antibody was used as the control and goat anti-mouse SC-2005 (Santa Cruz) or goat anti-rabbit SC-2004 (Santa Cruz) was used as the secondary antibody. The results are given in Figure 6 (B).

The results shown in Figures 6 and 7 obtained with HEK293 cells were similar to those obtained with NSC34 cells. In other words, alsin suppressed Tollip-induced cell death.

### (Example 4 Suppression of Tollip-induced cell death by the MORN domain of ALS2)

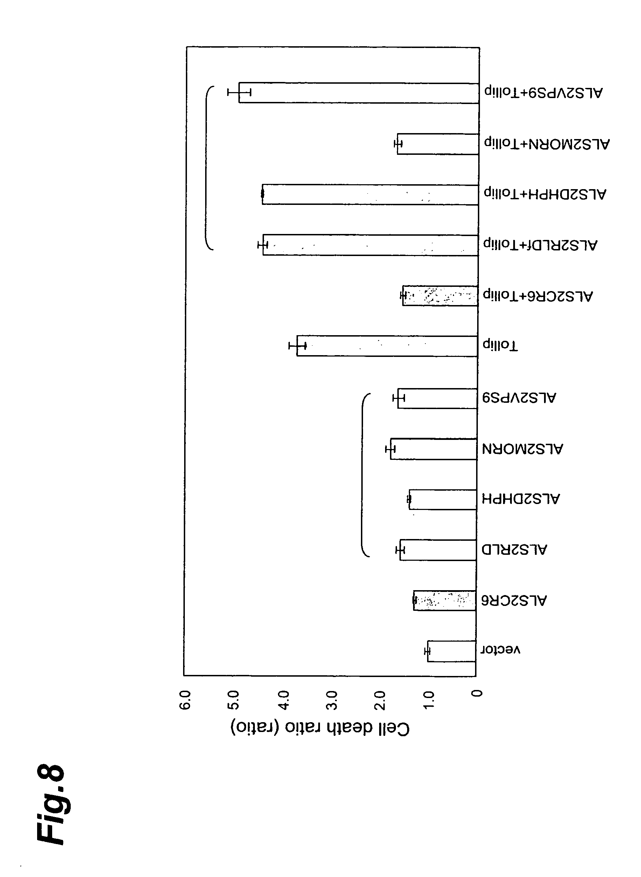

Whether Tollip-induced cell death was suppressed in NSC34 cells was checked for each domain of ALS2 by trypan blue staining. The method of the experiment was the same as in Example 3. Results are given in Figure 8. Figure 8 confirms that out of the four domains, only MORN had suppressive effect on Tollip-induced cell death.

### (Example 5 Effect of ALS2 on the NFκB-suppressing action of Tollip)

There have been reports of Tollip suppressing LPS-induced NFκB activity in IL-1R/TLR signaling. The present inventors therefore decided to examine whether ALS2 had any effect on the NFκB-suppressing action of Tollip. As the IL-1R/TLR signaling is complex, the present inventors used instead a system where TNF-α induced the NFκB activity to examine the effect of ALS2 on NFκB-suppressing action of Tollip.

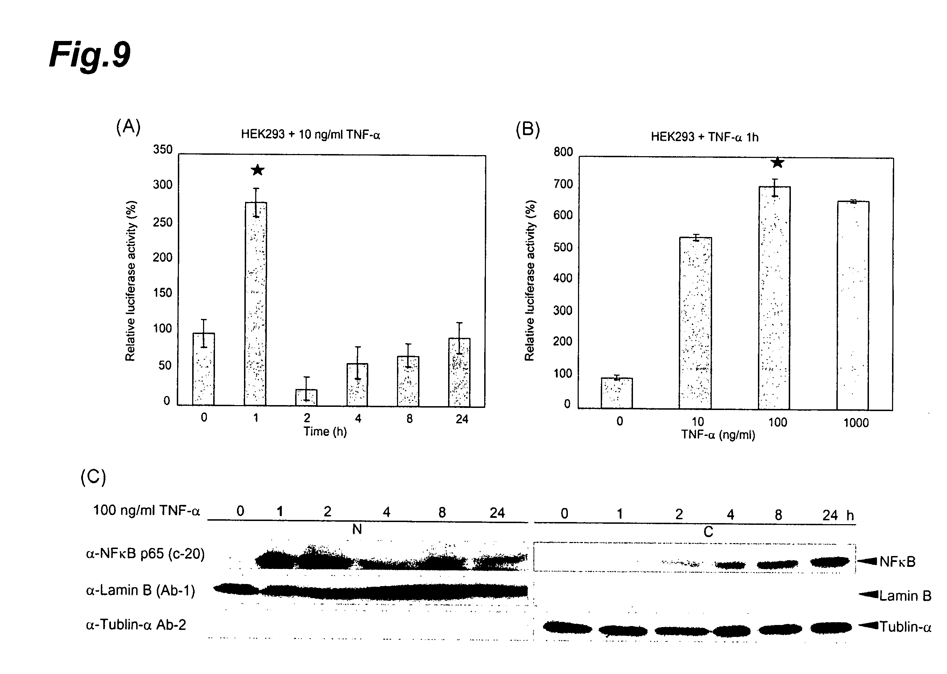

Firstly, for establishing the experiment system, we screened various treatment times and doses of TNF-α to determine the optimum conditions for induction of NFκB activity by TNF-α. The luciferase assay was used for the screening. To be more specific, firstly, the NFκB gene was introduced upstream of LUC into pELAM1-Luc, which is a luciferase (LUC) reporter vector, and a vector for expressing NFκB/LUC fusion protein was prepared. Next, HEK293 cells were transfected with this expression vector. 24 h after the transfection, the cells were treated with 10 ng/mL of TNF-α for 1, 2, 4, 8, or 24 h. After that, the luciferase assay was carried out by a standard method, and the level of transcription of the NFκB, which was the target gene, was examined. The results are given in Figure 9 (A). Figure 9 (A) confirms that highest NFκB activity was induced 1 h after the treatment with TNF-α.

The level of transcription of NFκB was measured using different concentrations of TNF-α: 10, 100, and 1000 ng/mL, keeping the treatment time constant at 1 h. The results are given in Figure 9 (B). Figure 9 (B) confirms that the highest NFκB activity was induced with 100 ng/mL.

Furthermore, the expression level of the NFκB protein in the cell nucleus and the cytoplasm of HEK293 cells treated with 100 ng/mL of TNF-α for 1, 2, 4, 8, or 24 h was examined using western blotting (WB). For this WB, rabbit anti-NFκB p65(C-20) SC-372 (Santa Cruz) was used as the primary antibody and goat anti-rabbit SC-2004 (Santa Cruz) as the secondary antibody. The results showed that the highest level of NFκB protein was seen in the cell nucleus 1 h after the treatment.

On the basis of the above results, we decided to use the optimal conditions for induction of NFκB activity by TNF-α, i.e., 1h of treatment with 100 ng/mL of TNF-α.

Next, the effect of ALS2 and Tollip on NFκB activity in TNF-α signaling was examined by the luciferase assay. HEK293 cells were co-transfected with pELAM1-Luc vector for expressing the above-described NFκB/LUC fusion protein, and ALS2CR6-FLAG-pIRESpuro2 and/or Tollip-Myc-pcDNA3, and treated 24 h later with 100 ng/mL of TNF-α for 1 h. After that, the luciferase assay was done by the standard method.

In preliminary tests, the effect of Tollip on NFκB activity was seen in cells treated with TNF-α 24 h or 48 h after transfection with Tollip. In the main experiment, the treatment with TNF-α was given 24 h after transfection, and various amounts of ALS2 or Tollip were transfected (dose of vector). The results are given in Figure 10 (A) and (B). Figure 10 (A) and (B) confirm that ALS induced NFκB activity in a dose-dependent manner, and that Tollip suppressed NFκB activity in a dose-dependent manner.

NFκB activity in HEK293 cells that had been transfected with ALS2 (187.5 ng) only, Tollip (187.5 ng) only, or both with ALS2 (187.5 ng) and Tollip (187.5 ng), and then treated for 1 h with 100 ng/mL of TNF-α, or not treated with it, was examined by the luciferase assay. The results are shown in Figure 10 (C). Figure 10 (C) confirms that ALS2 partially restored the NFκB activity suppressed by Tollip.

It was confirmed from the aforementioned results that Tollip suppressed NFκB activity in TNF-α signaling, as in IL-1R/TLR signaling, and that this suppressive action was inhibited by ALS2.

### (Example 6 Interaction between Tollip and IRAK-1)

There has been a report that Tollip binds to IRAK-1 in IL-1R/TLR signaling. We examined whether this happened in TNF-α signaling also. The experiment was carried out by the immunoprecipitation method, which can verify binding of proteins.

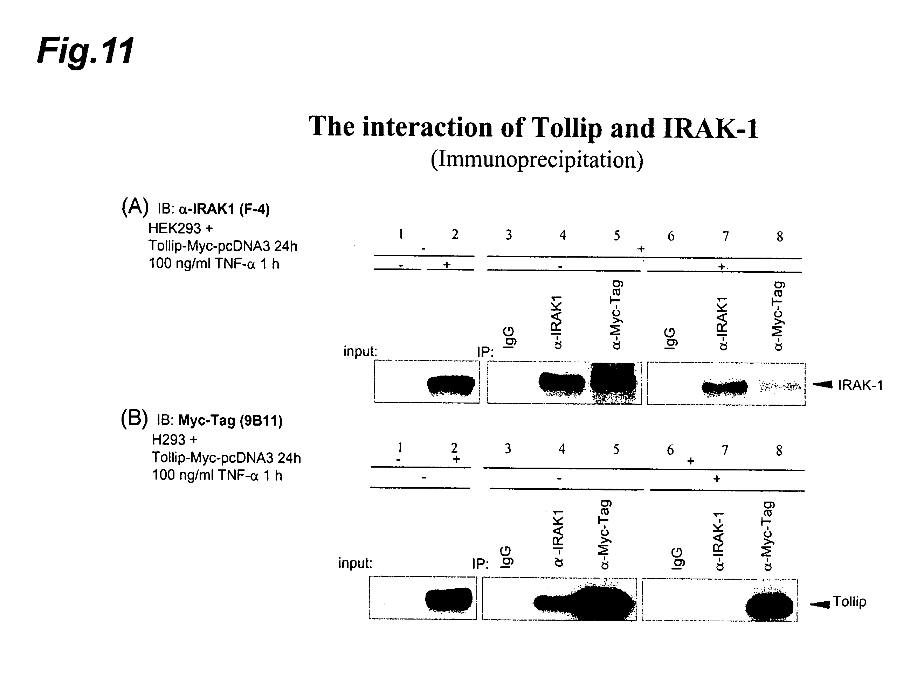

HEK293 cells were transfected with Tollip-Myc-pcDNA3. Then, after 24 h, some of the cells were treated for 1 h with 100 ng/mL of TNF-α while others were not treated. After that, the cells were lysed and immunoprecipitation (IB) was carried out. For the immunoprecipitation, Chrom PumPure mouse IgG (Jackson Immunoresearch) was used as the control, mouse anti-IRAK-1(F-4)SC-5288 (Santa Cruz) and mouse anti-Myc(9B11)#2276 (Santa Cruz) were used as primary antibodies, and goat anti-mouse SC-2005 (Santa Cruz) was used as the secondary antibody.
The results are given in Figure 11.

Figure 11 confirms that Tollip and IRAK-1 formed a complex by binding together (lane 5 of Figure 11 (A) and lane 4 of Figure 11 (B)), and that this binding was suppressed by treatment with TNF-α (lane 8 of Figure 11 (A) and lane 7 of Figure 11 (B)). From this, it can be concluded that Tollip and IRAK-1 bind to each other in TNF-α signaling as well. The results also suggest the possibility of Tollip suppressing NFκB activity through IRAK-1.

### (Example 7 TNF-α-mediated migration of Tollip to the cell nucleus)

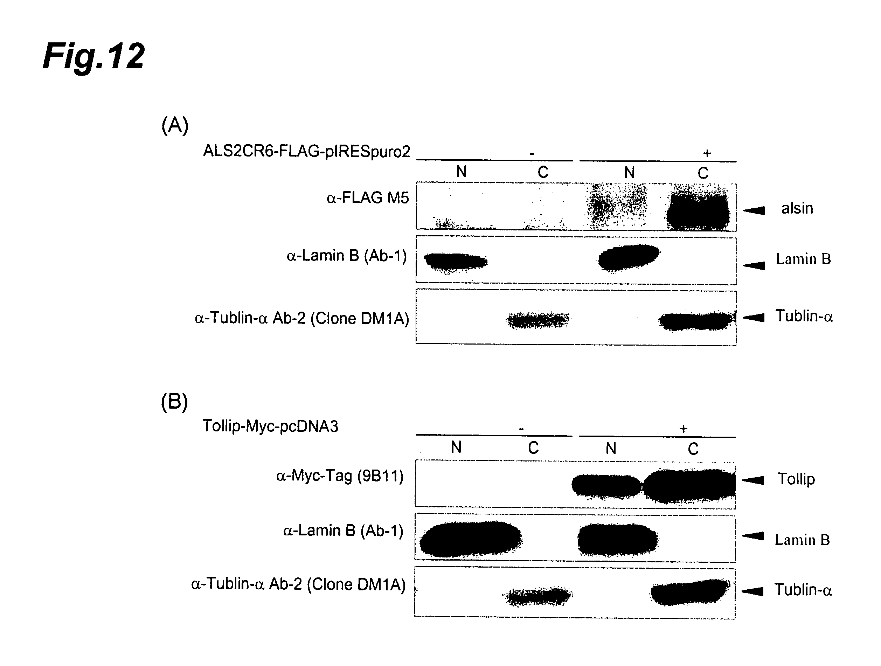

Firstly, the intracellular localization of alsin and Tollip was examined by cell fractionation and with western blotting. To be more specific, HEK293 cells were transfected with ALS2CR6-FLAG-pIRESpuro2 or Tollip-Myc-pcDNA3 and 24 h later, the cells were lysed and fractionated by a standard method. After that, western blotting (WB) was carried out. In the WB, lamin, which is localized in the cell nucleus only, and tubulin, which is localized in the cytoplasm only, were used as controls; mouse anti-lamin B (101-B7) NA12 (CALBIOCHEM) or mouse anti-tubulin (Ab-2) (clone DM1A) (LABVISION/NEOMARKERS) were used as primary antibodies, and goat anti-mouse SC-2005 (Santa Cruz) was used as the secondary antibody. The results are given in Figure 12.

Figure 12 confirms that alsin was almost entirely localized in the cytoplasm, whereas Tollip was present both in the cytoplasm and the cell nucleus.

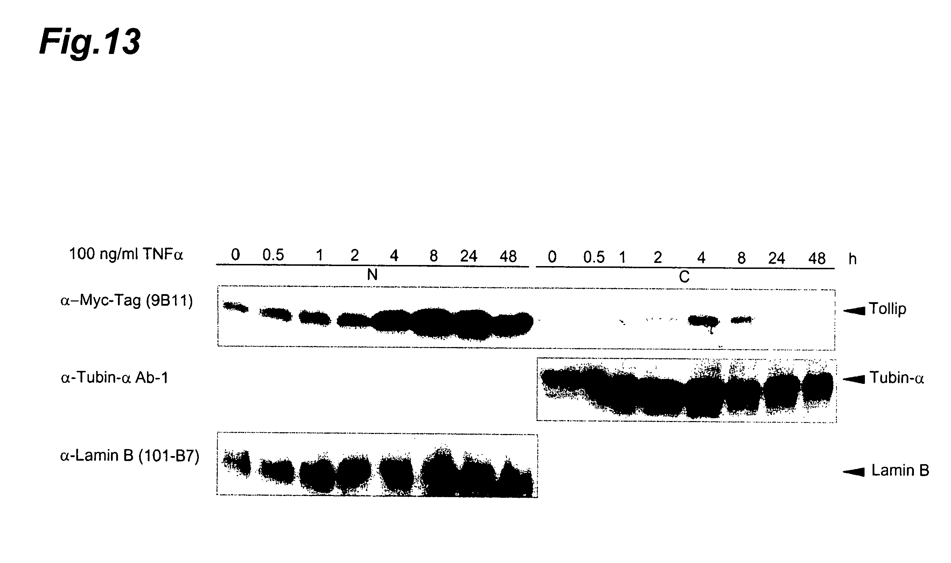

Next, with HEK293 cells transfected with Tollip-Myc-pcDNA3, we examined how the intracellular localization of Tollip changed by TNF-α treatment. Treatment with 100 ng/mL of TNF-α was given up to 48 h and changes in localization of Tollip were checked at different time points. The results are given in Figure 13. Figure 13 confirms that treatment with TNF-α decreased the expression of Tollip, and made Tollip to migrate to the cell nucleus (N).

### Industrial Applicability

Therapeutic agents for juvenile familial amyotrophic lateral sclerosis having a completely different action mechanism from what is known so far can be selected by using the screening method of the present invention. It is believed that therapeutic agents selected by the screening methods of the present invention will be promising new therapeutic agents for juvenile familial amyotrophic lateral sclerosis.

## Claims

1. A method of screening therapeutic agents for juvenile familial amyotrophic lateral sclerosis, comprising a step of assessing a substance that suppresses the expression of Tollip in cells as a therapeutic agent for juvenile familial amyotrophic lateral sclerosis.

2. A method of screening therapeutic agents for juvenile familial amyotrophic lateral sclerosis, comprising steps of:
culturing cells that express Tollip under the conditions of the presence and absence of a test substance;
measuring the level of Tollip expression in cells cultured under the different conditions; and
assessing the test substance as a therapeutic agent for juvenile familial amyotrophic lateral sclerosis if the level of Tollip expression in the cells cultured in the presence of the test substance is less than in the cells cultured in the absence of the test substance.

3. A method of screening therapeutic agents for juvenile familial amyotrophic lateral sclerosis, comprising a step of assessing a substance that promotes migration of Tollip in cells from the cytoplasm to the cell nucleus as a therapeutic agent for juvenile familial amyotrophic lateral sclerosis.

4. A method of screening therapeutic agents for juvenile familial amyotrophic lateral sclerosis, comprising steps of;
culturing cells that express Tollip under the conditions of the presence and absence of the test substance;
measuring the amount of Tollip present in the cytoplasm and the cell nuclei in cells cultured under the different conditions; and
assessing the test substance as a therapeutic agent for juvenile familial amyotrophic lateral sclerosis if the ratio of the amount of Tollip present in the cell nuclei, with respect to the total of the amount of Tollip present in the cytoplasm and the amount of Tollip present in the cell nuclei, is higher in the cells cultured in the presence of the test substance than in the cells cultured in the absence of the test substance.

5. A method of screening therapeutic agents for juvenile familial amyotrophic lateral sclerosis, comprising a step of assessing a substance that inhibits the interaction between Tollip and IRAK-1 in cells as a therapeutic agent for juvenile familial amyotrophic lateral sclerosis.

6. A method of screening therapeutic agents for juvenile familial amyotrophic lateral sclerosis, comprising steps of:
culturing cells that express Tollip and IRAK-1 under the conditions of the presence and absence of the test substance;
measuring the interaction between Tollip and IRAK-1 in the cells cultured under the different conditions; and
assessing the test substance as a therapeutic agent for juvenile familial amyotrophic lateral sclerosis if the interaction between Tollip and IRAK-1 is weaker in the cells cultured in the presence of the test substance than in the cells cultured in the absence of the test substance.

7. The method of screening therapeutic agents for juvenile familial amyotrophic lateral sclerosis according to any one of the claims 1 to 6, wherein ALS2 gene is a responsible gene for the juvenile familial amyotrophic lateral sclerosis.
